# EUROPÄISCHE PATENTSCHRIFT

(11) **EP 1 205 218 B1**
(45) Veröffentlichungstag und Bekanntmachung des Hinweises auf die Patenterteilung: **31.03.2004**
(21) Anmeldenummer: 00124790.7
(22) Anmeldetag: 14.11.2000
(51) Int. Cl.: A61P 17/06, A61K 45/06, A61K 31/57, A61K 31/17, A61K 31/473, A61K 31/47

(54) **Topical-Antipsoriaticum mit Harnstoff, einem Corticoid und einem Desinfektionsmittel**
Topical antipsoriaticum comprising urea, a corticoid and a desinfectant
Antipsoriatrique topical comprenant de l'urée, un corticoide et un désinfectant

(43) Veröffentlichungstag der Anmeldung: 15.05.2002
(73) Patentinhaber: Benkert, Eugen Georg, Dr.med.dent., 76532 Baden-Baden (DE)
(72) Erfinder: Benkert, Eugen Georg, Dr.med.dent., 76532 Baden-Baden (DE)
(74) Vertreter: Hellmayr, Wolfgang, Dr. rer. nat.

(56) Entgegenhaltungen:
- EP-A- 0 006 724
- EP-A- 0 224 837
- EP-A- 0 717 994
- WO-A-00/57845
- DE-A- 1 817 288
- GB-A- 2 116 425
- US-A- 4 367 224
- US-A- 4 512 978
- WOLF B ET AL: "UNTERSUCHUNGEN ZUR KOMPATIBILITAET VON WIRKSTOFFHALTIGEN DERMATIKA MIT ZUSAETZLICH EINGEARBEITETEN WIRKSTOFFEN" PHARMAZIE,DD,VEB VERLAG VOLK UND GESUNDHEIT. BERLIN, Bd. 51, Nr. 12, 1996, Seiten 966-970, XP000651797 ISSN: 0031-7144
- BUDAVARI ET AL: "The Merck Index 12th Edn." 1996 , MERCK & CO. INC. , WHITEHOUSE STATION NJ XP002167135 * Seite 115-116 *
- BRIEDE, R. H.: "Application of carbomer-water gel 1%" PHARM. WEEKBL. (1983), 118(9), 170-4 , XP001001186

## Beschreibung

Die Erfindung betrifft ein neues Arzneimittel, nämlich ein Antipsoriaticum zur äußerlichen Behandlung von Psoriasis in Form einer Salbe oder Creme.

Psoriasis (Schuppenflechte) ist eine weit verbreitete, chronische, multifaktoriell genetisch determinierte Hautkrankheit. Die Ursache der Krankheit liegt in einer Störung des Stoffwechsels der oberen Hautschichten. Sie ist durch unkontrolliertes, aber nicht malignes Wachstum des Epithels der Haut gekennzeichnet.

Zur Behandlung werden äußerlich u. a. gegebenenfalls halogenierte Corticoide (A) und Kombinationen mit Antiseptika und Teerprodukte sowie Bestrahlung mit UV-A nach einer Behandlung mit Psoralen-Präparaten angewendet.

So sind antipsoriatische Salben vorgeschlagen worden, die in einer Salbengrundlage als Wirkstoff Cortisone enthalten.

Andererseits sind auch Hautsalben oder -cremes, die hautverträgliche Desinfektionsmittel (B) zur Bekämpfung von pathogenen Mikroorganismen, wie Bakterien, Viren oder Kleinpilzen, enthalten, als Antipsoriatica vorgeschlagen und in der Therapie verwendet worden. Beispielhaft für solche Desinfektionsmittel seien hier 5-Chlor-7-iod-8-chinolinol (Clioquinol, Summenformel: C9 H5 C1 I N O) und Triclosan {5-Chlor-2-(2,4-dichlorphenoxy)-phenol} genannt.

Obwohl mit den bekannten Antipsoriatica gewisse Heilerfolge erzielt werden können, ist ihre Wirkung in vielen Fällen nur gering und/oder nur vorübergehend, oder sie sprechen bei manchen Patienten gar nicht an, was schon daran zu erkennen ist, daß 1 bis 2 % der Bevölkerung an Psoriasis leidet. Bei vielen Patienten muß die Therapie über Jahre oder Jahrzehnte hin fortgesetzt werden, um eine gewisse Linderung herbeizuführen. Aber eine Heilung konnte in vielen Fällen nicht erreicht werden. Es besteht daher ein Bedarf an einem neuen, besser wirksamen Antipsoriaticum.

Die Aufgabe, die der vorliegenden Erfindung zugrunde lag, war also die Bereitstellung eines leicht anwendbaren Arzneimittels. Eine weitere Aufgabe der Erfindung war die Bereitstellung eines überlegenen, bei möglichst vielen Erkrankten wirksamen, und zwar dauerhaft wirksamen Arzneimittels zur Psoriasisbehandlung.

Diese Aufgaben werden durch die Bereitstellung des neuen erfindungsgemäßen Arzneimittels, insbesondere eines Antipsoriaticums, gelöst.

Gegenstand der Erfindung ist nun ein Arzneimittel, enthaltend einen antipsoriatischen Wirkstoff in einer Salben- oder Creme-Grundlage, das dadurch gekennzeichnet ist, daß es als Wirkstoff eine Kombination aus (A) mindestens einem gegebenenfalls halogenierten Corticoid (Corticosteroid, z. B. Glucocorticosteroid), (B) mindestens einem hautverträglichen Desinfektionsmittel und (C) Harnstoff enthält.

Obwohl mit den bekannten Antipsoriatica gewisse Heilerfolge erzielt werden können, ist ihre Wirkung in vielen Fällen nur gering und/oder nur vorübergehend, oder sie sprechen bei manchen Patienten gar nicht an, was schon daran zu erkennen ist, daß 1 bis 2 % der Bevölkerung an Psoriasis leidet. Bei vielen Patienten muß die Therapie über Jahre oder Jahrzehnte hin fortgesetzt werden, um eine gewisse Linderung herbeizuführen. Aber eine Heilung konnte in vielen Fällen nicht erreicht werden. Es besteht daher ein Bedarf an einem neuen, besser wirksamen Antipsoriaticum.

Die Aufgabe, die der vorliegenden Erfindung zugrunde lag, war also die Bereitstellung eines leicht anwendbaren Arzneimittels. Eine weitere Aufgabe der Erfindung war die Bereitstellung eines überlegenen, bei möglichst vielen Erkrankten wirksamen, und zwar dauerhaft wirksamen Arzneimittels zur Psoriasisbehandlung.

Diese Aufgaben werden durch die Bereitstellung des neuen erfindungsgemäßen Arzneimittels, insbesondere eines Antipsoriaticums, gelöst.

Gegenstand der Erfindung ist nun ein Arzneimittel, enthaltend einen antipsoriatischen Wirkstoff in einer Salben- oder Creme-Grundlage, das dadurch gekennzeichnet ist, daß es als Wirkstoff eine Kombination aus (A) mindestens einem gegebenenfalls halogenierten Corticoid (Corticosteroid, z. B. Glucocorticosteroid), (B) mindestens einem hautverträglichen Desinfektionsmittel aus der Gruppe Clioquinol, Rivanol ® und Triclosan und (C) Harnstoff enthält.

Halogenierte Corticoide, insbesondere chlorierte und fluorierte, werden als Komponente (A) erfindungsgemäß im allgemeinen bevorzugt. Zu ihnen gehören beispielsweise
Mometason-furoat [Mometason ist ein internationaler Freiname [i.F.] für Pregna-1,4-dien-3,20-dion-9,21-dichloro-17-{(2-furanylcarbonyl)oxy}-11-hydroxy-16-methyl-(11β,16α)-(9C1), Summenformel: C27 H30 C12 O6],
Betamethason-17-valerat (Betamethason ist ein i.F. für 9α-Fluor-11β,17α ,21-trihydroxy-16β-methyl-1,4-pregnadien-3,20-dion; Summenformel: C22 H29 F O5),
Triamcinolon-acetonid (Triamcinolon ist ein i.F. für 9-Fluor-11β,16α,17,21-tetrahydroxy-1,4-pregnadien-3,20-dion; Summenformel: C21 H27 F O6),
Dexamethason (i.F.für 9α-Fluor-11β,17,21-trihydroxy-16α-methyl-pregna-1,4-dien-3,20-dion; Summenformel: C22 H29 F O5),
Flumethason-21-pivalat (Flumethason ist ein i.F. für 6α,9-Difluor-11β,17,21-trihydroxy-16α-methyl-1,4-pregnadien-3,20-dion; Summenformel: C22 H28 F2 O5),
Flupredniden-21-acetal (Flupredniden ist ein i.F. für 9-Fluor-11β,17,21-trhydroxy-16-methylen-1,4-pregnadien-3,20-dion; Summenformel: C22 H27 F O5),
Amcinonid (i.F. für das Glukocorticoid 16α,17α-Cyclopentylidendioxy-9α-fluor-11β,21-dihydroxy-1,4-pregnadien-3,20-dion-21-acetat; Summenformel: C28 H35 F O7)
Diflorason-17,21-diacetat (Diflorason ist ein i.F. für das Glucocorticoid 6α,9-Difluor-11β,17,21-trihydroxy-16β-methyl-1,4-pregnadien-3,20-dion; Summenformel: C22 H28 F2 O5),
Clobetasol-17-propionat (21-Chlor-9-fluor-17-hydroxy-16β-methyl-1,4-pregnadien-3, 11, 20-trion),
Clocortolon (9-Chlor-6αfluor-11β,21-dihydroxy-16α-methyl-1,4-pregnadien-3,20-dion) und
Fluocinolonacetonid (6α,9-Difluor-11β-dihydroxy-16α,17-isopropylidendioxid).

Erfindungsgemäß geeignete, nicht halogenierte Corticoide sind beispielsweise
Hydrocortison (11β, 17, 21-Trihydroxy-4-pregnen-3,20-dion),
Prednison (i.F. für 17α,21-Dihydroxy-1,4-pregnadien-3,11,20-trion [1-Dehydrocortison], Summenformel: C21 H26 O5) und
Prednisolon (i.F. für 11β,17,21-Trihydroxy-1,4-pregnadien-3,20-dion [1-Dehydrohydrocortison], Summenformel: C21 H28 O5).

Erfindungsgemäß geignete, hautverträgliche Desinfektionsmittel sind 5-Chlor-7-iod-8-chinolinol (Clioquinol), Rivanol ® (ein Ethacridinderivat; Ethacridin ist ein i.F. für das desinfizierend wirksame 6,9-Diamino-2-ethoxyacridin, Summenformel: C15 H15 N3 O) und Triclosan.

Die Komponente (C) des erfindungsgemäßen Arzneimittels ist Harnstoff. Dieser wirkt, wie angenommen werden kann, vermutlich als antiseptisches Hautpflegemittel, Keratolytikum, Feuchtigkeitsspender, Lösungsvermittler und/oder Resorptionsbeschleuniger für lokal applizierte Wirkstoffe. Dem Harnstoff ist wegen seiner multifunktionalenWirkungen der Vorzug zu geben.

Als Salben- bezw. Creme-Grundlage sind übliche auf Kohlenwasserstoffgelen, Lipogelen (Wachsen, Fetten), Hydrogelen, Polyethylenglykolgelen und oder Silikongelen aufgebaute Grundlagen, die auch Stabilisatoren, Antioxidantien, Feuchthaltemittel u. s. w. enthalten können, geeignet.
A) Eine geeignete Creme-Grundlage (Basiscreme) nach DAC 99 (Deutcher Arzneimittel-Codex) setzt sich folgendermaßen zusammen:

| | |
|---|---|
| 4,0 g | Glycerolmonostearat |
| 6,0 g | Cetylalkohol |
| 7,5 g | mittelkettige Triglyceride |
| 25,5 g | weiße Vaseline |
| 7,0 g | Macrogol-1000-glycerolmonostearat |
| 10,0 g | Propylenglykol |
| 40,0 g | Wasser |
| 100,0 | |

B) Eine geeignete Salben-Grundlage (Basissalbe) besteht beispielsweise, bezogen auf 1 g, aus 950 mg weißem Vaselin und 50 mg dickflüssigem Paraffin.
C) Ein andere beispielhaft genannte Creme-Grundlage enthält auf 1 g:

| | |
|---|---|
| 150 mg | weiße Vaseline |
| 60 mg | dickflüssiges Paraffin |
| 72 mg | Cetylstearylalkohol |
| 22,5 mg | Cetomacrogol 1000 |
| 3 mg | Natriumhydrogenphosphat |
| 1 mg | Chlorocresol |
| Rest: | gereinigtes Wasser |

Die Mischungsverhältnisse der Bestandteile der neuen antipsoriatischen Wirkstoffkombination liegen im allgemeinen zwischen 0,025 bis 0,5 Gew.% (A), 1 bis 5 Gew.% (B) und 1 bis 10 Gew% (C), Rest Salben- bezw. Creme-Grundlage. Bevorzugt sind Mischungsverhältnisse von 0,05 bis 0,25 Gew.% (A), 2 bis 3,5 Gew.% (B), und 2 bis 7 Gew% (C), Rest Salben- bezw. Creme-Grundlage. Besonders bevorzugt verwendet man beipielsweise 1 bis 2 Gew% im Falle, daß Triclosan als (B) verwendet wird.

Beim Ansetzen der Rezepturen ist zu berücksichtigen, daß manche Corticoide sich in ihrer spezifischen Wirksamkeit graduell erheblich unterscheiden.

Die äußerliche Anwendung des erfindungsgemäßen, neuen Arzneimittels führt zu in überraschender Weise überlegenen, nachhaltigen Heilerfolgen in der Psoriasistherapie. Die Ergebnisse sind wesentlich besser als sie mit bekannten Präparaten, die einen der Komponenten (A) bis (C) alleine oder nur zwei der Komponenten (A) bis (C) enthalten, zu erzielen sind. Der mit dem neuen Heilmittel erhaltene Synergieeffekt war überraschend.

Die Erfindung wird nachstehend an Hand von Ausführungsbeispielen näher veranschaulich.

### Beispiel 1

1 g Mometason-furoat (Mometason ist ein i.F. für Pregna-1,4-dien-3,20-dion, 9,21-dichloro-17-{(2-furanylcarbonyl)oxy}-11-hydroxy-16-methyl-,(11β,16α)-(9Cl), 20g Clioquinol und 30g Harnstoff, Rest auf 1 kg insgesamt an Basis-Creme nach DAC (s. oben) wurden miteinander im Rührwerk bei Raumtemperatur innig vermischt, indem die Bestandteile zunächst in eine kleine Portion Basis-Creme eingerührt wurden und dann die übrige Basis-Creme portionsweise unter Rühren zugesetzt wurde. Das Produkt wurde in Dosen gefüllt und abgekühlt.

### Beispiel 2

1,5 g Betamethason-17-valerat (Betamethason ist ein i.F. für 9α-Fluor-11β,17α ,21-trihydroxy-16β -methyl-1,4-pregnadien-3,20-dion; Summenformel: C22 H29 F O5), 20 g Rivanol ® (ein Ethacridinderivat; Ethacridin ist ein i.F. für das desinfizierend wirksame 6,9-Diamino-2-ethoxyacridin, Summenformel: C15 H15 N3 O) und 25 g Harnstoff, Rest auf 1 kg insgesamt an Basis-Creme nach DAC (s. oben) wurden miteinander im Rührwerk bei etwa 30 Grad C. innig vermischt, in Dosen gefüllt und abgekühlt.

### Beispiel 3

In ähnlicher Weise wie in den vorstehend beschriebenen Ausführungsbeispielen wurden
250 mg Dexamethason-21-isonicotinat (9-Fluor-16 -methyl-prednisolon)
10 g Triclosan und
50 g Harnstoff auf 1 Kg Basis-Creme
zu einem Antipsoriaticum verarbeitet.

## Patentansprüche

1. Arzneimittel, enthaltend einen antipsoriatischen Wirkstoff in einer Salben- oder Creme-Grundlage, **dadurch gekennzeichnet, daß** es als Wirkstoff eine Kombination aus (A) mindestens einem Corticoid, (B) mindestens einem hautverträglichen Desinfektionsmittel aus der Gruppe Clioquinol, Rivanol ® und Triclosan und (C) Harnstoff enthält.

2. Arzneimittel nach Anspruch 1, **dadurch gekennzeichnet, daß** es als (A) halogeniertes Corticoid enthält.

3. Arzneimittel nach Anspruch 1 und/oder 2, **dadurch gekennzeichnet, daß** es als (A) Mometason-furoat [Mometason ist ein internationaler Freiname [i.F.] für Pregna-1,4-dien-3,20-dion-9,21-dichloro-17-{(2-furanylcarbonyl)oxy}-11-hydroxy-16-methyl-(11β,16α)-(9Cl), Summenformel: C27 H30 C12 O6],
Betamethason-17-valerat (Betamethason ist ein i.F. für 9α-Fluor-11β,17α ,21-trihydroxy-16β -methyl-1,4-pregnadien-3,20-dion; Summenformel: C22 H29 F O5),
Triamcinolon-acetonid (Triamcinolon ist ein i.F. für 9-Fluor-11β,16α,17,21-tetrahydroxy-1,4-pregnadien-3,20-dion; Summenformel: C21 H27 F O6),
Dexamethason (i.F.für 9α-Fluor-11β,17,21-trihydroxy-16α-methyl-pregna-1,4-dien-3,20-dion; Summenformel: C22 H29 F O5),
Flumethason-21-pivalat (Flumethason ist ein i.F. für 6α,9-Difluor-11β,17,21-trihydroxy-16α-methyl-1,4-pregnadien-3,20-dion; Summenformel: C22 H28 F2 O5),
Flupredniden-21-acetal (Flupredniden ist ein i.F. für 9-Fluor-11β,17,21-trhydroxy-16-methylen-1,4-pregnadien-3,20-dion ; Summenformel: C22 H27 F O5),
Amcinonid (i.F. für das Glukocorticoid 16α,17α-Cyclopentylidendioxy-9α-fluor-11β,21-dihydroxy-1,4-pregnadien-3,20-dion-21-acetat, Summenformel: C28 H35 F O7),
Diflorason-17,21-diacetat (Diflorason ist ein i.F. für das Glucocorticoid 6α,9-Difluor-11β,17,21-trihydroxy-16β-methyl-1,4-pregnadien-3,20-dion; Summenformel: C22 H28 F2 O5),
Clobetasol-17-propionat (21-Chlor-9-fluor-17-hydroxy-16β-methyl-1,4-pregnadien-3,11,20-trion),
Clocortolon (9-Chlor-6αfluor-11β,21-dihydroxy-16α-methyl-1,4-pregnadien-3,20-dion),
Fluocinolonacetonid (6α,9-Difluor-11β-dihydroxy-16α,17-isopropylidendioxid),
Hydrocortison (11β,17,21-Trihydroxy-4-pregnen-3,20-dion),
Prednison (i.F. für 17α,21-Dihydroxy-1,4-pregnadien-3,11,20-trion [1-Dehydrocortison], Summenformel: C21 H26 O5) und/oder
Prednisolon (i.F. für 11β,17,21-Trihydroxy-1,4-pregnadien-3,20-dion [1-Dehydrohydrocortison], Summenformel: C21 H28 O5)
enthält.

4. Arzneimittel nach einem der Ansprüche 1 bis 3, **dadurch gekennzeichnet, daß** es 0,025 bis 0,5 Gew.% (A), 1 bis 5 Gew.% (B), und 1 bis 10 Gew% (C), Rest Salben- bezw. Creme-Grundlage, enthält.

5. Arzneimittel nach einem der Ansprüche 1 bis 4, **dadurch gekennzeichnet, daß** es 0,05 bis 0,25 Gew.% (A), 2 bis 3,5 Gew.% (B), und 2 bis 7 Gew% (C), Rest Salben- bezw. Creme-Grundlage, enthält.

6. Verwendung des Arzneimittels nach einem der Ansprüche 1 bis 5 zur Herstellung eines Antipsoriaticums.

## Claims

1. A drug containing an antipsoriatic active substance in an ointment or cream basis, **characterized in that** it contains as an active substance a combination of (A) at least one corticoid hormone, (B) at least one skin-friendly disinfectant from the group of clioquinole, Rivanol ® and Triclosane and, (C) a urea.

2. The drug according to claim 1, **characterized in that** it contains halogenated corticoid as (A).

3. The drug according to claim 1 and/or 2, **characterized in that** it contains as (A) Mometasone Fuorate [Mometasone is an international nonproprietary name for 1,4-pregnadien-3,20-dione-9,21-dichloro-17-{2-furanylcarbonyl)oxy}-11-hydroxy-16-methyl-(12β,16α)-(9Cl); sum formula: C27 H30 C12 O6],
Betamethasone-17-Valerate (Bethametasone is an international nonproprietary name for 9α-fluoro-11β,17α,21-trihydroxy-16β-methyl-1,4-pregnadien-3,20-dione; sum formula: C22 H29 F O5),
Dexamethasone (international nonproprietary name for 9α-fluoro-11β,17,21-trihydroxy-16α-methyl-1,4-pregnadien-3,20-dione; sum formula: C22 H29 F O5),
Diflorasone-17,21-Diacetate (Diflorasone is an international nonproprietary name for the glucocorticoid 6α,9-difluoro-11β,17,21-trihydroxy-16β-methyl-1,4-pregnadien-3,20-dione; sum formula: C22 H28 F2 O5),
Clobetasole-17-Propionate (21-chloro-9-fluoro-17-hydroxy-16β-methyl-1,4-pregnadien-3,11,20-trione),
Clocortolone (9-chloro-6αfluoro-11β,21-dihydroxy-16α-methyl-1,4-pregnadien-3,20-dione),
Fluoccinolone Acetonide (6α,9-difluoro-11β-dihydroxy-16α,17-isopropylidenedioxide),
Prednisolone (international nonproprietary name for 11β,17,21-trihydroxy-1,4-pregnadien-3,20-dione [1-dehydrohydrocortisone]; sum formula: C21 H28 O5).

4. The drug according to one of the claims 1 to 3, **characterized in that** it contains 0.025 to 0.5 weight % (A), 1 to 5 weight % (B), and 1 to 10 weight % (C), residual ointment or cream basis.

5. The drug according to one of the claims 1 to 4, **characterized in that** it contains 0.05 to 0.25 weight % (A), 2 to 3.5 weight % (B), and 2 to 7 weight % (C) of the residual ointment or cream basis.

6. A use of the drug according to one of the claims 1 to 5 for the preparation of an antipsoriaticum.

## Revendications

1. Médicament contenant une substance active antipsoriatique dans une base de crème ou d'onguent, **caractérisé en ce qu'**on utilise comme substance active une combinaison de (A) au moins un corticoïde, (B) au moins un moyen désinfectant supporté par la peau du groupe Clioquinol, Rivanol® et Triclosan et (C) et de l'urée.

2. Médicament selon la revendication 1, **caractérisé en ce qu'**il contient en (A) du corticoïde halogéné.

3. Médicament selon la revendication 1 et/ou 2, **caractérisé en ce qu'**il contient en (A) du mométasonefuroate [mométasone dans une dénomination commune internationale (DCI) du prégna-1,4-diène-3,20-dione-9,21-dichloro-17-{(2-furanylocarbonyl)oxy}-11-hydroxy-16-méthyle]-(11β,16α)-(9C1), formule cumulée : C27 H30 C12 06],
du bétaméthasone-17-valérate (le bétaméthasone est une DCI du 9α-fluor-11β,17α,21-trihydroxy-16β-méthyle-1,4-prégnadiène-3,20-dione ; formule cumulée : C22 H29 F 05,
du déxaméthasone (DCI du 9α-fluor-11β,17,21-trihydroxy-16α-méthyle-prégna-1,4-diène-3,20-dione ; formule cumulée : C22 H29 F 05),
du diflorasone-17,21-diacétate (le diflorasone est une DCI du glucocorticoïde 6α,9-difluor-11β, 17,21-trihydroxy-16β-méthyle-1,4-prégnadiène-3,20-dione ; formule cumulée : C22 H28 F2 05),
du clobétasol-17-propionate (21-chlor-9-fluor-17-hydroxy-16β-méthyle-1,4-prégnadiène-3,11,20-trione),
du Clocortolon (9-chlor-6αfluor-11β,21-dihydroxy-16α-méthyle-1,4-prégnadiène-3,20-dione),
du fluocinolonacétonide (6α,9-difluor-11β-dihydroxy-16α,17-isopropylidènedioxyde),
du Prednisolon (DCI du 11α,17,21-trihydroxy-1,4-prégnadiène-3,20-dione [1-déhydrohydrocortisone], formule cumulée : C21 H28 05).

4. Médicament selon l'une quelconque des revendications 1 à 3, **caractérisé en ce qu'**il contient de 0,025 à 0,5 % en poids de (A), 1 à 5 % en poids de (B), et 1 à 10 % en poids de (C), le reste étant la base d'onguent ou de crème.

5. Médicament selon l'une quelconque des revendications 1 à 4, **caractérisé en ce qu'**il contient de 0,05 à 0,25 % en poids de (A), 2 à 3,5 % en poids de (B), et 2 à 7 % en poids de (C), le reste étant la base d'onguent ou de crème.

6. Utilisation du médicament selon l'une quelconque des revendications 1 à 5 pour la préparation d'un antipsoriatique.
